(19) Europäisches Patentamt
European Patent Office
Office européen des brevets

(11) EP 2 574 347 B1

(12) EUROPEAN PATENT SPECIFICATION

(45) Date of publication and mention
of the grant of the patent:
**16.05.2018 Bulletin 2018/20**

(51) Int Cl.:
*A61K 51/00* *(2006.01)*     *A61K 51/12* *(2006.01)*
*A61K 51/02* *(2006.01)*     *A61P 35/00* *(2006.01)*

(21) Application number: **12193933.4**

(22) Date of filing: **08.03.2004**

(54) **Microspheres comprising therapeutic and diagnostic radioactive isotopes**

Mikrokügelchen mit therapeutischen und diagnostischen radioaktiven Isotopen

Microspheres contenant des isotopes radioactifs therapeutiques et diagnostiques

(84) Designated Contracting States:
**AT BE BG CH CY CZ DE DK EE ES FI FR GB GR
HU IE IT LI LU MC NL PL PT RO SE SI SK TR**

(30) Priority: **04.04.2003 US 407144**

(43) Date of publication of application:
**03.04.2013 Bulletin 2013/14**

(62) Document number(s) of the earlier application(s) in
accordance with Art. 76 EPC:
**04809310.8 / 1 615 671**

(73) Proprietor: **BIOSPHERE MEDICAL, INC.**
**South Jordan, UT 84095 (US)**

(72) Inventors:
• **Schwarz, Alexander**
**Brookline, MA 02446 (US)**
• **Krom, James, A**
**Belmont, MA 02478 (US)**

(74) Representative: **Bennett, Nicholas**
**Cleveland**
**10 Fetter Lane**
**London EC4A 1BR (GB)**

(56) References cited:
**WO-A-02/34300     US-A- 5 302 369**

• R.J. MUMPER ET AL: "Neutron-Activated
Holmium-166-Poly (L-Lactic Acid) Microspheres:
A Potential Agent for the Internal Radiation
Therapy of Hepatic Tumors", JOURNAL OF
NUCLEAR MEDICINE, vol. 32, no. 11, November
1991 (1991-11), pages 2139-2143, XP002531706,

• **U.O. HÄFELI ET AL: "Hepatic Tumor
Radioembolization in a Rat Model Using
Radioactive Rhenium (186RE/188RE) Glass
Microspheres", INT. J. RADIATION ONCOLOGY
BIOL. PHYS., vol. 44, no. 1, April 1999 (1999-04),
pages 189-199, XP002531707,**
• **NIJSEN J F W ET AL: "Advances in nuclear
oncology: microspheres from internal
radionuclide therapy of liver tumors", 1 January
2002 (2002-01-01), CURRENT MEDICINAL
CHEMISTRY, BENTHAM SCIENCE PUBLISHERS
BV, BE, PAGE(S) 73 - 82, XP002983145, ISSN:
0929-8673 * the whole document ***
• **LUDWIG E. FEINENDEGEN: "Editorial -
Microdosimetric Considerations of Hepatic
Radioembolization", THE JOURNAL OF
NUCLEAR MEDICINE, vol. 35, no. 10, 1 October
1994 (1994-10-01), pages 1644-1646,
XP055063777,**
• **JAMES C. ANDREWS ET AL: "Hepatic
Radioembolization with Yttrium-90 Containing
Glass Microspheres: Preliminary Results and
Clinical Follow-Up", THE JOURNAL OF
NUCLEAR MEDICINE, vol. 35, no. 10, October
1994 (1994-10), pages 1637-1644, XP055063780,**
• **S. HO ET AL: "Clinical Evaluation of the Partition
Model for Estimating Radiation Doses from
Yttrium-90 Microspheres in the Treatment of
Hepatic Cancer", EUROPEAN JOURNAL OF
NUCLEAR MEDICINE, vol. 24, no. 3, March 1997
(1997-03), pages 293-298, XP002531708,**
• **ARIEL I M ET AL: "Therapeutic intralymphatic
infusion of radioactive isotopes", 1 January 1963
(1963-01-01), JOURNAL OF NUCLEAR
MEDICINE, SOCIETY OF NUCLEAR MEDICINE,
RESTON, VA, US, PAGE(S) 186, XP008106947,
ISSN: 0161-5505 * Abstract D1 ***

**Description**

BACKGROUND OF THE INVENTION

The development of new and more effective treatments for cancer is of utmost concern. This is particularly relevant for the treatment of malignant tumors found in the liver owing to the current unsatisfactory treatment options. At the present time, the preferred method of treatment for patients with liver metastases is surgical resection. Unfortunately, the 5-year survival rate for patients that have undergone this form of treatment is only around 35%. Scheele J and Altendorf-Hofmann A. Resection of colorectal liver metastases. Langenbeck's Arch. Surg. 1999; 313-327. This disappointingly low survival rate is compounded by the fact that most tumours are inoperable by the time of diagnosis. Other treatment options for these tumours include conventional chemotherapy and external radiotherapy. Häfeli U O, Casillas S, Dietz D W, Pauer G J, Rybicki L A, Conzone S D and Day D E. Hepatic tumor radioembolization in a rat model using radioactive rhenium (186Re/188Re) glass microspheres. Int. J. Radiation Oncology Biol. Phys. 1999; 44:189-199 and Link K H, Komnman M., Formentini A, Leder G, Sunelaitis E, Schatz M, Prelmar J and Beger H G. Regional chemotherapy of non-resectable liver metastases from colorectal cancer-literature and institutional review. Langenbeck's Arch. Surg. 1999; 384:344-353. Unfortunately, neither of the latter regimens have shown significant improvements in patient survival.

Recent developments in selective radionuclide therapy indicate that radiolabeled microspheres may offer a promising treatment option for patients suffering from a variety of types of cancer. This treatment allows the selective delivery of therapeutic radioactive particles to the tumor with as little surrounding tissue damage as possible. This new treatment option is particularly important for cancers with an extremely poor prognosis and without other adequate therapies, such as primary and metastatic malignancies of the liver. For example, the regional administration of therapeutic agents via the hepatic artery is one strategy that has been developed to improve tumour response. Bastian P, Bartkowski R, Kohler H and Kissel T. Chemo-embolization of experimental liver metastases. Part 1: distribution of biodegradable microspheres of different sizes in an animal model for the locoregional therapy. Eur. J. Pharm. Biopharm. 1998; 46:243-254. This form of treatment promises to be particularly effective for both primary and metastatic liver cancer since these tumors are well vascularized and receive the bulk of their blood supply from the hepatic artery. Ackerman N B, Lien W M, Kondi E S and Silverman N A. The blood supply of experimental liver metastases. The distribution of hepatic artery and portal vein blood to "small" and "large" tumors. Surgery 1969; 66:1067-1072. In addition, many kinds of radiolabeled particles and radionuclides have been tested for local treatment of a variety of tumors in organs, including liver, lung, tongue, spleen and soft tissue of extremities.

In early applications of this technique, yttrium oxide powder was suspended in a viscous medium prior to administration. Yttrium oxide was selected for the technique because it emits nearly 100 percent beta radiation. See Nolan et al., Intravascular Particulate Radioisotope Therapy, The American Surgeon 1969; 35: 181-188 and Grady et al., Intra-Arterial Radioisotopes to Treat Cancer, American Surgeon 1960; 26:678-684. However, the yttrium oxide powder had a high density (5.01 gm/cm$^3$) and irregular particle shape. The high density of pure yttrium oxide powder made it difficult to keep the particles in suspension in the liquids used to inject them into the body, and the sharp corners and edges of yttrium oxide particles also irritate surrounding tissue in localized areas. In later applications, the particles used have been microspheres composed of an ion exchange resin, or crystalline ceramic core, coated with a radioactive isotope such as P-32 or Y-90. Both ion exchange resin and crystalline ceramic microspheres offer the advantage of having a density much lower than that of yttrium oxide particles, and the ion exchange resin offers the additional advantage of being particularly easy to label. See Zielinski and Kasprzyk, Synthesis and Quality Control Testing of 32P labelled Ion Exchange Resin Microspheres for Radiation Therapy of Hepatic Neoplasms, Int. J. Appl. Radiat. Isot. 1983; 34:1343-1350. In still another application, microspheres have been prepared comprising a ceramic material and having a radioactive isotope incorporated into the ceramic material. While the release of radioactive isotopes from a radioactive coating into other parts of the human body may be eliminated by incorporating the radioisotopes into ceramic spheres, the latter product form is nevertheless not without its disadvantages. Processing of these ceramic microspheres is complicated because potentially volatile radioactivity must be added to ceramic melts and the microspheres must be produced and sized while radioactive, with the concomitant hazards of exposure to personnel and danger of radioactive contamination of facilities.

The current technology often uses glass, resin, albumin, or polymer microspheres that are impregnated with a material that emits β-particles upon neutron activation. Research has indicated that the composition of the bead can be important in the design of an effective treatment. For example, glass is relatively resistant to radiation-damage, highly insoluble, and non-toxic. Glass can be easily spheridized in uniform sizes and has minimal radionuclidic impurities. Advances in manufacturing have led to the production of glass microspheres with practically no leaching of the radioactive material. Ho S, Lau W Y, Leung T W T, Chan M, Ngar Y K, Johnson P J and Li A K C. Clinical evaluation of the partition model for estimating radiation doses from yttrium-90 microspheres in the treatment of hepatic cancer. Eur. J. Nucl. Med. 1997; 24:293-298.

Although glass spheres have several advantages, their high density (3.29 g/ml) and non-biodegradability are major drawbacks. Mumper R J, Ryo U Y and Jay M. Neutron activated holmium-166-Poly(L-lactic acid) microspheres: A potential agent for the internal radiation therapy of hepatic tumours. J. Nucl. Med. 1991; 32:2139-2143 and Turner J H, Claringbold P G, Klemp P F B, Cameron P J, Martindale A A, Glancy R J, Norman P E, Hetherington E L, Najdovski L and Lambrecht R M. 166Ho-microsphere liver radiotherapy: a preclinical SPECT dosimetry study in the pig. Nucl. Med. Comm. 1994; 15:545-553. The relatively high density increases the chance of intravascular settling. Ho S, Lau W Y, Leung T W T and Johnson P J. Internal radiation therapy for patients with primary or metastatic hepatic cancer. Cancer 1998; 83:1894-1907. Nevertheless, glass microspheres produced under the name TheraSpheres® were the first registered microsphere product for internal radionuclide therapy, and have been used in patients with primary or metastatic tumours. In comparison, only a few radioisotopes have the characteristics necessary for the treatment of tumors. Important characteristics of a suitable radioisotope would include a radiational spectrum (strength of β-particle emision) appropriate to the size of the tumor, high dose rate, short half-life, and γ-emission for external imaging.

The most suitable radioactive materials are yttrium-90, rhenium-188 and holmium-166. All three of these materials emit β-radiation useful for radiotherapy. Although $^{90}$Y is often used in radionuclide therapy, yttrium-90 has two major disadvantages for use in radiotherapy. First, long neutron activation times (>2 weeks) are needed to achieve therapeutic activities of yttrium because $^{90}$Y's precursor has a small thermal neutron cross section of 1.28 barn. Secondly, the biodistribution of microspheres loaded with $^{90}$Y cannot be directly determined in clinical trials, since $^{90}$Y is a pure β-emitter and does not produce imageable γ-rays. Natural rhenium is composed of two isotopes, $^{185}$Re and $^{187}$Re, that form β-emitting $^{186}$Re and $^{188}$Re radioisotopes, respectively, upon neutron activation. The nuclear and dosimetric properties of the rhenium radioisotopes are comparable to those of $^{90}$Y, but they have imageable γ-photons. Like the rhenium radioisotopes, $^{166}$Ho emits β-particles and photons and has a relatively short physical half-life of 26.8 h, compared to $^{90}$Y (64.1 h) and $^{186}$Re (90.6 h), resulting in a high dose rate.

The development of microspheres for radionuclide therapy is complicated by the difficulty in determining the biodistribution of the microspheres in vivo, as noted above for $^{90}$Y. The biodistribution of microspheres is critically important for this type of radiotherapy because the microsphere must be in close proximity to the tumor being treated. One potential solution to this problem would be to attach a material to the microsphere that emits a detectable, non-hazardous signal.

## Summary of the Invention

One aspect of the present invention relates to a microsphere, comprising a material selected from the group consisting of polymer and resin; a first radioisotope that emits a therapeutic β-particle; and a second radioisotope that emits a diagnostic γ-ray; wherein the atomic number of the first radioisotope is not the same as the atomic number of the second radioisotope, according to the claims. The present invention also relates to a method of preparing a radioactive microsphere, comprising the steps of: combining a non-radioactive precursor of a first radioisotope, a non-radioactive precursor of a second radioisotope, and a material selected from the group consisting of polymer, and resin, to form a mixture; wherein the atomic number of the first radioisotope is not the same as the atomic number of the second radioisotope; fabricating a microsphere from said mixture; and bombarding said microsphere with neutrons, according to the claims. Another aspect of the present invention relates to a microsphere according to the claims for use in a method of treating a mammal suffering from a medical condition, comprising the step of administering to said mammal a therapeutically effective amount of radioactive microspheres each comprising a material selected from the group consisting of polymer, and resin; a first radioisotope that emits a therapeutic β-particle; and a second radioisotope that emits a diagnostic γ-ray; wherein the atomic number of the first radioisotope is not the same as the atomic number of the second radioisotope.

## Detailed Description of the Invention

The invention will be described more fully with reference to the accompanying examples, in which certain preferred embodiments of the invention are shown.

Radionuclide therapeutic techniques using microspheres for the treatment of various cancers rely upon the precise and accurate delivery of microspheres to a tumor. This treatment option offers the promise of delivering therapy directly to the tumor cells which minimizes damage to nearby healthy tissue, a serious shortcoming associated with conventional treatment options such as chemotherapy, radiotherapy, or surgical resection. However, the effectiveness of cancer treatments using radionuclide microspheres is often hampered by the inability to determine the biodistribution of the microspheres. Therefore, a non-invasive method to determine the biodistribution of said microspheres would be highly useful. Microspheres containing $^{198}$Au, for emission of γ-radiation to enable detection, have been designed that incorporate $^{90}$Y for emission of β-particles useful in the treatment of various medical conditions. A method to determine the amount of $^{197}$Au required per microsphere has been established based on the physical properties and relative

proportions of $^{90}Y$, $^{198}Au$, and the bulk material comprising the bead. A mathematical formula has been derived that allows for the calculation of the necessary quantity of the stable isotope of the therapeutic β-emitting radionuclide and $^{197}Au$. The composition and size of the microsphere may be customized to best fit a particular application. The radiolabeled microspheres may be introduced into a subject mammal in accord with standard procedures and the biodistribution of the microspheres may be determined by detection of the gamma-rays emitted by $^{198}Au$.

*Derivation of the Rate of Radioactive Decay*

Neutron activation of a stable isotope to give a radioactive isotope is described by the simple scheme where $k_N$ and $k_D$ are the neutron capture constant and radioactive decay constant, respectively.

$$A \xrightarrow{k_N} A^* \xrightarrow{k_D} P$$

A, $A^*$, and $P$ represent the numbers of atoms (or moles) of the stable isotope, radioactive isotope, and decay product, respectively. The net rate of formation of the radioactive isotope $A^*$ is given by

$$\frac{dA^*}{dt} = k_N A - k_D A^* \qquad (Eq\ 1)$$

which has the solution, assuming no A* is present initially,

$$A^* = \frac{k_N A_0}{k_D - k_N}(e^{-k_N t} - e^{-k_D t}) \qquad (Eq\ 2)$$

where $A_0$ is the initial quantity of the stable isotope.

The radioactive decay constant can be expressed in terms of the radioactive isotope's half-life ($t_{1/2}$) according to:

$$k_D = \frac{\ln(2)}{t_{1/2}} \qquad (Eq\ 3)$$

The neutron capture constant is determined by the neutron flux φ and the neutron capture cross-section χ according to:

$$k_N = \varphi\chi \qquad (Eq\ 4)$$

Constants φ and χ are typically expressed in units of cm$^{-2}$s$^{-1}$ and $10^{-24}$ cm$^2$ (barns), respectively.

After removing the material from the neutron source, the radioactive isotope will decay at a rate given by

$$\frac{dA^*}{dt} = -k_D A^* \qquad (Eq\ 5)$$

*Determination of Required Quantity of Stable Isotope*

The radioactivity, defined by $-dA^*/dt$, is usually expressed in units of s$^{-1}$ (becquerel) or 3.7 x $10^{10}$ s$^{-1}$ (curie). From Eq 2 and Eq 5, the equation that expresses the radioactivity at the time of removal of the sample from the neutron source is

$$\frac{-dA^*}{dt} = \frac{k_D k_N A_0 (e^{-k_N t} - e^{-k_D t})}{k_D - k_N} \qquad (Eq\ 6)$$

Solving Eq 6 for $A_0$ allows calculation of the quantity of stable isotope that is required to achieve a desired radioactivity after an irradiation time t according to:

$$A_0 = \frac{dA^*}{dt} \frac{k_D - k_N}{k_D k_N (e^{-k_N t} - e^{-k_D t})} \qquad (Eq\ 7)$$

*Identity of β-Emitting Therapeutic Radionuclide*

A radionuclide suitable for internal radionuclide therapy of primary and metastatic malignancies must have the following properties: First, the radioisotope must have an appropriate radiation spectrum for treating small to large multiple tumours. Large tumours with a vascular periphery but a necrotic centre take up less microspheres per volume; therefore, a high energy β-emitter with a subsequently high tissue range is needed to reach the interior of the tumour. Second, a high dose rate is advantageous for the radiobiological effect. Spencer R P. Applied principles of radiopharmaceutical use in therapy. Nucl. Med. Biol. 1986; 13:461-463 and Spencer R P. Short-lived radionuclides in therapy. Nucl. Med. Biol. 1987; 14:537-538. Consequently, a short half-life is preferable. Third, a γ-emitter is desirable for external imaging to determine the biodistribution of the radioisotope with a gamma camera. However, the radioactivity should be low to prevent unnecessary radiation burden to the patient and environment. Mumper R J, Ryo U Y and Jay M. Neutron activated holmium-166-Poly(L-lactic acid) microspheres: A potential agent for the internal radiation therapy of hepatic tumours. J. Nucl. Med. 1991; 32:2139-2143. Further, the labeling of particles has to be simple without any leakage of the isotope. Finally, a large thermal neutron cross section is needed to enable high specific activities to be achieved within short neutron activation times. Conzone S D, Häfeli U O, Day D E and Ehrhardt G J. Preparation and properties of radioactive rhenium glass microspheres intended for in vivo radioembolization therapy. J. Biomed. Mater. Res. 1998; 42:617-625. Unfortunately, only a few radioisotopes have characteristics which make them potentially suitable for the treatment of tumours. Suitable radionuclides are selected from the group consisting of $^{90}$Y, $^{99m}$Tc, $^{188}$Re, $^{32}$P, $^{166}$Ho, $^{109}$Pd, $^{140}$La, $^{153}$Sm, $^{165}$Dy, and $^{169}$Er. In preferred embodiments, the radionuclide is $^{90}$Y, $^{166}$Ho, or $^{188}$Re.

*Detection of γ Photons Emitted by Diagnostic Radionuclide*

Today, cancer is often found using a gamma camera, which provides images of potential tumors in the body by detecting the radiation emitted by a radiopharmaceutical given to a patient undergoing a full-body scan. In such systemic approaches, suspected tumor regions collect higher concentrations of the radiopharmaceutical, which produces a higher count rate and therefore a detectable contrast between the tumor region and its surroundings.

Most clinically-used radiopharmaceuticals are diagnostic agents incorporating a gamma-emitting nuclide which, because of physical or metabolic properties of its coordinated ligands, localizes in a specific organ after intravenous injection. The resultant images can reflect organ structure or function. These images are obtained by means of a gamma camera that detects the distribution of ionizing radiation emitted by the radioactive molecules. The principal isotope currently used in clinical diagnostic nuclear medicine is metastable technetium-99m, which has a half-life of 6 hours.

As outlined above, a gamma camera is used in nuclear medicine for the display, in an organ, of the distribution of molecules marked by a radioactive isotope injected into a patient. Thus, a gamma camera has a collimator to focus the gamma photons emitted by the patient's body, a scintillator crystal to convert the gamma photons into light photons or scintillations, and an array of photomultiplier tubes, each of which converts the scintillations into electrical pulses. A detection system such as this is followed by a processing and display unit that can be used to obtain an image projection of the distribution of the radioactive isotopes in the patient during the acquisition of the image.

*Activation of Radionuclide*

A variety of neutron sources, such as reactors, accelerators, and radioisotopic neutron emitters, can be used for radioactivation of stable isotopes by neutron activation. Systems and methods for neutron activation are described in U.S. Pat. Nos. 6,149,889 and 6,328,700. Nuclear reactors with their high fluxes of neutrons from uranium fission offer the highest available activation rates for most elements. Different types of reactors and different positions within a reactor vary considerably with regard to their neutron energy distributions and fluxes due to the materials used to moderate (or reduce the energies of) the primary fission neutrons. However, most neutron energy distributions are quite broad and include three principal components (thermal, epithermal, and fast).

The thermal neutron component includes low-energy neutrons (energies below 0.5 eV) in thermal equilibrium with atoms in the reactor's moderator. At room temperature, the energy spectrum of thermal neutrons is best described by a Maxwell-Boltzmann distribution with a mean energy of 0.025 eV and a most probable velocity of 2200 m/s. In most reactor irradiation positions, 90-95% of the neutrons that bombard a sample are thermal neutrons.

The epithermal neutron component includes neutrons (energies from 0.5 eV to about 0.5 MeV) which have been only partially moderated. A cadmium foil 1 mm thick absorbs all thermal neutrons, but will allow epithermal and fast neutrons above 0.5 eV in energy to pass through. In a typical unshielded reactor irradiation position, the epithermal neutron flux represents about 2% the total neutron flux. Both thermal and epithermal neutrons induce reactions on target nuclei.

The fast neutron component of the neutron spectrum (energies above 0.5 MeV) includes the primary fission neutrons which still have much of their original energy following fission. Fast neutrons contribute very little to the reaction, but instead induce nuclear reactions where the ejection of one or more nuclear particles-(n,p), (n,n'), and (n,2n)-are prevalent. In a typical reactor irradiation position, about 5% of the total flux consists of fast neutrons.

The amount of radiation delivered to a target by radioactive metal or metal compound labeled microspheres can be controlled in a variety of ways; for example, by varying the amount of metal associated with the spheres, the extent of radioactivation of the metal, the quantity of microspheres administered, and the size of microspheres administered.

*Bulk Composition of Microspheres*

*Glass*

Glass is relatively resistant to radiation-damage, highly insoluble, and non-toxic. Glass can be easily spheridized in uniform sizes and has minimal radionuclidic impurities. Advances in technology have led to the production of glass microspheres with practically no leaching of radioactive material. Ho S, Lau W Y, Leung T W T, Chan M, Ngar Y K, Johnson P J and Li A K C. Clinical evaluation of the partition model for estimating radiation doses from yttrium-90 microspheres in the treatment of hepatic cancer. Eur. J Nucl. Med. 1997; 24:293-298. Although the glass spheres have several advantages, their high density (3.29 g/ml) and their non-biodegradability are drawbacks. Mumper R J, Ryo U Y and Jay M. Neutron activated holmium-166-Poly(L-lactic acid) microspheres: A potential agent for the internal radiation therapy of hepatic tumours. J. Nucl. Med. 1991; 32:2139-2143. The relatively high density of glass increases the chance of intravascular settling. Glass microspheres produced under the name TheraSpheres® were the first registered microsphere product for internal radionuclide therapy, and are used in patients with primary or metastatic tumours. Because of the lack of $\gamma$-emission of $^{90}$Y, radioactive rhenium ($^{186}$Re/$^{188}$Re) microspheres were also produced. The general method of manufacture of these spheres was the same as for the $^{90}$Y spheres. Häfeli U O, Casillas S, Dietz D W, Pauer G J, Rybicki L A, Conzone S D and Day D E. Hepatic tumor radioembolization in a rat model using radioactive rhenium (186Re/188Re) glass microspheres. Int. J. Radiation Oncology Biol. Phys. 1999; 44:189-199 and Conzone S D, Häfeli U O, Day D E and Ehrhardt G J. Preparation and properties of radioactive rhenium glass microspheres intended for in vivo radioembolization therapy. J. Biomed. Mater. Res. 1998; 42:617-625.

Brown et al. prepared $^{166}$Ho-loaded glass particles for direct injection into tumours of mice, which resulted in an effective modality for deposition of intense $\gamma$-radiation for use in localized internal radionuclide therapy; however, no further studies were done. Brown R F, Lindesmith L C and Day D E. 166-Holmium-containing glass for internal radiotherapy of tumors. Int. J. Rad. Appl. Instrum. B 1991; 18:783-790.

Kawashita et al. suggested the use of phosphorus-rich $Y_2O_3$-$Al_2O_3$-$SiO_2$-glass microspheres containing phosphorus ions, which were produced by thermoelectron bombardment of red phosphorus vapour and implanted into glass, thus resulting in a high phosphorus content and high chemical durability. After activation by neutron bombardment the glass contains phosphorus-32 ($^{32}$P). Kawashita M, Miyaji F, Kokubo T, Takaoka G H, Yamada I, Suzuki Y and Inoue M. Surface structure and chemical durability of P<+> - implanted Y2O3-Al2O3-SiO2 glass for radiotherapy of cancer. J. Non-Cryst. Solids 1999; 255:140-148.

*Resins*

Resin-based microspheres are favoured for radio-embolization. Chloride salts of holmium and yttrium have been added to cation exchange resins. Different resins were investigated by Schubiger et al., amongst which were Bio-Rex 70, Cellex-P, Chelex 100, Sephadex S P and A G 50W-X8. Schubiger P A, Beer H-F, Geiger L, Rösler H, Zimmerman A, Triller J, Mettler D and Schilt, W. 90Y-resin particles-animal experiments on pigs with regard to the introduction of superselective embolization therapy. Nucl. Med. Biol. 1991; 18:305-311. The resins with $^{90}$Y bound to the carboxylic acid groups of the acrylic polymer were sterilized and used for renal embolization of pigs. Only the pre-treated Bio-Rex 70 resulted in usable particles, with a retention of beta activity in the target organ of >95% of injected dose, and no histologically detectable particles in lung tissue samples. Zimmerman A, Schubiger P A, Mettler D, Geiger L, Triller J and Rösler H. Renal pathology after arterial yttrium-90 microsphere administration in pigs. A model for superselective radioembolization therapy. Invest. Rad. 1995; 30:716-723.

Aminex resins (Bio-Rad Inc. Hercules Calif., USA) loaded with $^{166}$Ho or $^{188}$Re also resulted in usable preparations. Turner et al. prepared microspheres by addition of $^{166}$Ho-chloride to the cation exchange resin Aminex A-5, which has sulphonic acid functional groups attached to styrene divinylbenzene copolymer lattices. Turner J H, Claringbold P G, Klemp P F B, Cameron P J, Martindale A A, Glancy R J, Norman P E, Hetherington E L, Najdovski L and Lambrecht R M. 166Ho-microsphere liver radiotherapy: a preclinical SPECT dosimetry study in the pig. Nucl. Med. Comm. 1994; 15:545-553. Reproducible, non-uniform distributions of the $^{166}$Ho-microspheres throughout the liver were observed on scintigraphic images, following intrahepatic arterial administration in pigs. This predictable distribution allowed these investigators to determine the radiation absorbed dose from a tracer activity of $^{166}$Ho-microspheres, and to define the administered activity required to provide a therapeutic dose. Aminex A-27 was labelled with $^{188}$Re by adding $^{188}$Re-perrhenate and $SnCl_2$ to vacuum-dried resin particles. Wang S-J, Lin W-Y, Chen M.-N, Chi C-S, Chen J-T, Ho W-L, Hsieh B-T, Shen L-H, Tsai Z-T, Ting G, Mirzadeh S and Knapp F F. Intratumoral injection of rhenium-18 8microspheres into an animal model of hepatoma. J Nucl. Med. 1998; 39:1752-1757. The mixture was boiled and centrifuged and microspheres were separated and resuspended in saline. Spheres were tested by direct intratumoural injection into rats with hepatoma. Survival over 60 days was significantly better in the treated versus the control group (80% vs. 27%).

Investigators from Australia and Hong Kong have used unspecified resin-based particles labeled with $^{90}$Y for treatment of patients with primary or secondary liver cancer. Lau W Y, Leung W T, Ho S, Leung N W Y, Chan M, Lin J, Metreweli C, Johnson P and Li A K C. Treatment of inoperable hepatocellular carcinoma with intrahepatic arterial yttrium-90 microspheres: a phase I and II study. Br. J. Cancer 1994; 70:994-999. The spheres had a diameter of 29-35 $\mu$m, a density of 1.6 g/mL and a specific activity of approximately 30-50 Bq per sphere. Treatment was well tolerated with no bone-marrow or pulmonary toxicity. The median survival was 9.4 months (range 1.8-46.4) in 71 patients, and the objective response rate in terms of drop in tumour marker levels was higher than that based on reduction in tumour volume shown by computed tomography. Lau W Y, Ho S, Leung T W T, Chan M, Ho R, Johnson P J and Li A K C. Selective internal radiation therapy for nonresectable hepatocellular carcinoma with intraarterial infusion of 90yttrium microspheres. Int. J. Radiation Oncology Biol. Phys. 1998; 40:583592.

Albumin

Since 1969, technetium-99m-microspheres ($^{99m}$Tc-microspheres) of human serum albumin (HSA) have been widely used for clinical nuclear medicine, particularly for lung scanning. Wunderlich G, Pinkert J, Andreeff M, Stintz M, Knapp F F, Kropp J and Franke W G. Preparation and biodistribution of rhenium-188 labeled albumin microspheres B 20: a promising new agent for radiotherapy. Appl. Radiat. Isotopes 2000; 52:63-68 and Rhodes B A, Zölle I, Buchanan J W and Wagner H N. Radioactive albumin microspheres for studies of the pulmonary circulation. Radiology 1969; 92:1453-1460. $^{188}$Re-labeled HSA microspheres used by Wunderlich et al. are uniform in size, with a mean diameter of 25 $\mu$m, and are biocompatible and biodegradable. However, the labeling process is time-consuming and depends on $SnCl_2 2H_2O$ and gentisic acid concentration. On the surface of the microspheres a shell of less than about 1 $\mu$m thickness was seen, probably consisting of precipitated tin hydroxide. The particle labeling (coating) may be achieved by a combination of the reduction reaction of RE(VII) with Sn(II) and a particle surface-related coprecipitation effect of tin hydroxide colloid with high adsorption capacity and reduced, hydrolysed rhenium. The labeling yield under optimal reaction conditions is more than 70%. Biodistribution experiments in rats, using the lungs as a model for a well-perfused tumour, resulted in excellent in vivo stability.

As well as rhenium, yttrium has been bound to HSA for internal radiotherapy. Watanabe N, Oriuchi N, Endo K, Inoue T, Tanada S, Murata H and Sasaki Y. Yttrium-90 labeled human macroaggregated albumin for internal radiotherapy: combined use with DTPA. Nucl. Med. Biol. 1999; 26:847-851. $^{90}$Y-acetate and macroaggregates of HSA (MAA) (Macrokit®, Dainabot, Tokyo, Japan) were suspended in sodium acetate buffer and incubated at room temperature. Experiments in mice were carried out in order to investigate the possibility of using $^{90}$Y-MAA as an internal radiotherapeutic agent for whole-lung irradiation. Yttrium-activity in the lung was cleared within 72 h post injection and activity was redistributed in other organs, especially in the bone, but this could be prevented by the combined use of $CaNa_3DTPA$. Based on its rapid clearance $^{90}$Y-MAA was suggested as being useful for fractionated internal radiotherapy of the lung.

*Polymers*

Polymer-based microspheres have many advantages over other materials, in particular their near-plasma density, biodegradability and biocompatibility. However, the major disadvantage is their inability to withstand high thermal neutron fluxes. Conzone S D, Häfeli U O, Day D E and Ehrhardt G J. Preparation and properties of radioactive rhenium glass microspheres intended for in vivo radioembolization therapy. J. Biomed. Mater. Res. 1998; 42:617-625. Additives and adjustment of irradiation-parameters can overcome this problem. A solvent evaporation technique has been used for preparation of poly(L-lactic acid) (PLLA) microspheres containing $^{166}$Ho, $^{90}$Y and $^{186}$Re/[88]Re. Mumper et al. has prepared PLLA microspheres with holmium-165-acetylacetonate (HoAcAc). Mumper R J and Jay M. Poly(L-lactic acid) microspheres containing neutron-activatableholmium-165: A study of the physical characteristics of microspheres before and after irradiation in a nuclear reactor. Pharm. Res. 1992; 9:149-154. HoAcAc complex and PLLA were dissolved in chloroform and the solution was added to a polyvinyl alcohol (PVA) solution and stirred until the solvent had evaporated. Microspheres were graded and collected according to size, on stainless steel sieves having 20-50 $\mu$m openings. These microspheres can be dispensed in patient-ready doses that only need to be activated by neutron bombardment to a therapeutic amount of radioactivity in a nuclear reactor. These holmium loaded microspheres are currently being tested by intrahepatic arterial administration to rat liver tumours. A seven-fold increase of the $^{166}$Ho microspheres in and around the tumour compared with normal liver was found, based on distribution of radioactivity.

Magnetic PLLA microspheres loaded with yttrium were made by Hafeli et al. in order to direct them to the tumour. Häfeli U O, Sweeney S M, Beresford B A, Humm J L and Macklis R M. Effective targeting of magnetic radioactive 90Y-microspheres to tumor cells by an externally applied magnetic field. Preliminary in vitro and in vivo results. Nucl. Med. Biol. 1995; 22:147-155. This method resulted in stably loaded spheres, with the possibility of pre- or afterloading. To produce preloaded microspheres, PLLA was dissolved with L-[alpha]-phosphatidylcholine in methylene chloride. Commercially available $^{90}$YCl$_3$ and magnetite Fe$_3$O$_4$ were added to the solution, vortexed, and sonicated. The suspension was injected into PBS with PVA, and microspheres were prepared following a solvent evaporation technique. Afterloaded spheres were prepared by suspending dried microspheres in a solution of PBS, after which $^{90}$YCl$_3$ in HCl was added. Spheres were subsequently vortexed, incubated, and washed, resulting in labeled microspheres. Leaching of $^{90}$Y was around 4% after 1 day in PBS at 37°C. Specific activity was 1.85 MBq/mg in both methods. $^{90}$Y was bound to the carboxylic endgroups of the PLLA. Experiments in mice showed a 12-fold increase in activity in the tumour with a directional magnet fixed above it. Rhenium loaded PLLA microspheres were also developed, but these microspheres were unable to withstand the high neutron fluxes in a nuclear reactor which are necessary to achieve the high specific activity required in the treatment of liver tumours. Häfeli U O, Casillas S, Dietz D W, Pauer G J, Rybicki L A, Conzone S D and Day D E. Hepatic tumor radioembolization in a rat model using radioactive rhenium (186Re/188Re) glass microspheres. Int. J. Radiation Oncology Biol. Phys. 1999; 44:189-199.

*Manufacture of Microspheres*

In certain cases, such as described in U.S. Pat. No. 5,302,369, microspheres have been prepared from a homogenous mixture of powders (i.e., the batch) that is melted to form the desired glass composition. The exact chemical compounds or raw materials used for the batch is not critical so long as they provide the necessary oxides in the correct proportion for the melt composition being prepared. For instance, if a YAS glass is being made, then yttria, alumina, and silica powders could be used as the batch raw materials. The purity of each raw material is preferably greater than 99.9%. After either dry or wet mixing of the powders to achieve a homogeneous mixture, the mixture may be placed in a platinum crucible for melting. High purity alumina crucibles can also be used if at least small amounts of alumina can be tolerated in the glass being made. The crucibles containing the powdered batch are then placed in an electric furnace which is heated 1500° to 1600° C., depending upon the composition. In this temperature range, the batch melts to form a liquid which is stirred several times to decrease its chemical heterogeneity. The melt should remain at 1500° to 1600° C. until all solid material in the batch is totally dissolved, usually 2-5 hours being sufficient. When melting and stirring is complete, the crucible is removed from the furnace and the melt is quickly quenched to a glass by pouring the melt onto a cold steel plate or into clean water. This procedure breaks the glass into fragments, which aids and simplifies crushing the glass to a fine powder. The powder is then sized and spheroidized for use.

Where it is desired to use microspheres having a diameter in the range of about 20 to about 30 micrometers, as for in the treatment of liver cancer, it is preferred that the quenched and broken glass be first crushed to about minus 100 mesh particles using a mortar and pestle. The minus 100 mesh material is then ground using a mechanized mortar and pestle or ball mill, until it passes a 400 mesh sieve. The particles are formed into glass microspheres by introducing the -400 mesh particles into a gas/oxygen flame where they are melted and a spherical liquid droplet is formed by surface tension. The droplets are rapidly cooled before they touch any solid object so that, their spherical shape is retained in the solid product.

Just prior to spheroidizing, the -400 mesh powder is rescreened through a 400 mesh sieve to remove any large agglomerates that may have formed during storage. The -400 mesh powder is then placed in a vibratory feeder located above the gas/oxygen burner. The powder is slowly vibrated into a vertical glass tube which guides the falling powder particles directly into the hot flame of a gas/oxygen burner. Any burner capable of melting - 400 mesh particles of the particular glass composition being used is satisfactory. A typical rate for feeding the powder to the flame is 5 to 25 gm/hr with the described apparatus. The flame of the burner is directed into a metal container which catches the small glass beads as they are expelled from the flame. This container can be made of any metal which can withstand the heat of the burner and does not contaminate the glass. The container needs to be large enough so that the molten spheres can cool and become rigid before hitting a solid surface.

After spheroidization, the glass spheres are collected and rescreened. When the microspheres are intended to be used in the treatment of liver cancer, the fraction less than 30 and greater than 20 micrometers in diameter is recovered since this is the desirable size for use in the human liver. After screening, the -30/+20 microspheres are examined with an optical microscope and are then washed with a weakly acidic solution, filtered, and washed several times with reagent grade acetone. The washed spheres are then heated in a furnace in air to 500°-600° C. for 2-6 hours to destroy any organic material.

The final step is to examine a representative sample of the -30/+20 spheres in a scanning electron microscope to evaluate the size range and shape of the spheres. The quantity of undersize spheres (less than 10 micrometers in diameter) is determined along with the concentration of non-spherical particles. The composition of the spheres can be checked by energy dispersive x-ray analysis to confirm that the composition is correct and that there is an absence of chemical contamination. The glass microspheres are then ready for irradiation and subsequent administration to the patient.

Polymer-based microspheres used for internal radionuclide therapy are mainly prepared by a solvent evaporation technique. In the solvent evaporation process, the polymer is dissolved in a suitable water immiscible volatile solvent, and the medicament is dispersed or dissolved in this polymeric solution. The resulting solution or dispersion is then emulsified by stirring in an aqueous continuous phase, thereby forming discrete droplets. In order that the microspheres should form, the organic solvent must first diffuse into the aqueous phase and then evaporate at the water/air interface. As solvent evaporation occurs the microspheres harden, and free flowing microspheres can be obtained after suitable filtration and drying. O'Donnell P B and McGinity J W. Preparation of microspheres by solvent evaporation technique. Adv. Drug Del. Rev. 1997; 28:25-42

*Administration of Microspheres*

The microspheres may be administered to the patient through the use of catheters either alone or in combination with vasoconstricting agents or by any other means of administration that effectively causes the microspheres to become embedded in the cancerous or tumor bearing tissue. See U.S. Pat. No. 5,302,369. For purposes of administration, the microspheres are preferably suspended in a medium that has a sufficient density or viscosity that prevents the microspheres from settling out of suspension during the administration procedure. Presently, preferred liquid vehicles for suspension of the microspheres include polyvinylpyrrolidone (PVP), sold under the trade designation Plasdone K-30 and Povidone by GAF Corp, a contrast media sold under the trade designation Metrizamide by Nyegard & Co. of Oslo, Norway, a contrast media sold under the trade designation Renografin 76 by E. R. Squibb & Co., 50% dextrose solutions and saline.

*Selected Clinical Applications of Radionuclide Microspheres*

Given the increased skills of interventional radiologists, there is increasing interest in selective radionuclide therapy. Many kinds of radiolabeled particles and radionuclides have been tested for local treatment of a variety of tumours in organs, including liver, lung, tongue, spleen and soft tissue of extremities. The purpose of this treatment is the superselective application of suitable radioactive (high energetic β-emitters) particles to deliver high doses to the tumour, with as little surrounding tissue damage as possible. These new treatment methods are promising particularly for cancers with a poor prognosis and without other adequate therapies, such as primary and metastatic malignancies of the liver.

*Liver Cancer*

Patients with primary or metastatic tumours were treated by radio-embolization via a catheter or direct injection of beads into the tumour with a needle. Gray B N, Burton M A, Kelleher D, Klemp P and Matz L. Tolerance of the liver to the effects of yttrium-90 radiation. Int. J. Radiation Oncology Biol. Phys. 1990; 18:619-623 and Tian J-H, Xu B-X, Zhang J-M, Dong B-W, Liang P and Wang X-D. Ultrasoundguided internal radiotherapy using yttrium-90-glass microspheres for liver malignancies. J. Nucl. Med. 1996; 37:958-963. Most studies describe administration of microspheres to patients via a catheter, whereby the tip was placed in the hepatic artery. The spheres eventually lodge in the microvasculature of the liver and tumour, remaining until the complete decay of the radioisotope. Lung shunting and tumour-to-normal liver ratio was determined after infusion of $^{99m}$Tc-labeled macroaggregated albumin, and microspheres were subsequently administered to patients. Ho S, Lau W Y, Leung T W T, Chan M, Chan K W, Lee W Y, Johnson P J and Li A K C. Tumour-to-normal ratio of 90Y microspheres in hepatic cancer assessed with 99mTc macroaggregated albumin. Brit. J. Rad. 1997; 70:823-828. Tumour-to-normal liver ratio was approximately 3-5. Yorke E D, Jackson A, Fox R A, Wessels B W and Gray N. Can current models explain the lack of liver complications in Y-90 microsphere therapy? Clin. Cancer Res. 1999; 5:3024s-3030s. In some studies the blood flow within the liver was temporarily redirected in favour of the tumour by a bolus infusion of a vasoconstrictor, and the spheres were then embolized into the arterial circulation. While external beam radiation causes radiation hepatitis at doses above 30-35 Gy the liver can tolerate up to 80-150 Gy, using internal radionuclide therapy. Ingold J, Reed G, Kaplan H and Bagshaw M. Radiation hepatitis. Am. J. Roentgenol. Radium Ther. Nucl. Med. 1965; 93:200-208. Increased longevity, pain relief, tumour response and total clinical improvement are frequently reported.

*Head and Neck Cancers*

Chemo-embolization with ethylcellulose microspheres of 100-450 $\mu$m has been used in the treatment of maxillary tumours. The role of intra-arterial radioisotope therapy in the treatment of head and neck cancer is just beginning in rabbits, in the work of van Es et al. Van Es R J J, Franssen O, Dullens H F J, Bemsen M R, Bosman F, Hennink W E and Slootweg P J. The VX2 carcinoma in the rabbit auricle as an experimental model for intra-arterial embolization of head neck squamous cell carcinoma with hydrogel dextran microspheres. Lab. Anim. 1999; 33:175-184. The optimal size of microspheres for treatment of unresectable head-and-neck cancer is still to be established. Some embolizations in the treatment of head-and-neck cancer have been carried out with particles of 100-450 pm. Tomura N, Kato K, Hirano H, Hirano Y and Watarai J. Chemoembolization of maxillary tumors via the superficial temporal artery using a coaxial catheter system. Radiation Med. 1998; 16:157.

*Other Cancers*

Intra-arterial administration of $^{90}$Y-microspheres has been carried out in the spleen. Ariel I M and Padula G. Irradiation of the spleen by the intra-arterial administration of 90yttrium microspheres in patients with malignant lymphoma. Cancer 1972; 31:90-96. Of nine patients with lymphosarcoma, five manifested no clinical response after splenic irradiation. One patient who complained of weakness, rapid fatigue and anorexia, had relief of all symptoms after splenic irradiation

*Definitions*

For convenience, certain terms employed in the specification, examples, and appended claims are collected here.
The articles "a" and "an" are used herein to refer to one or to more than one (i.e. to at least one) of the grammatical object of the article. By way of example, "an element" means one element or more than one element.
The term "radionuclide" refers to a radioactive isotope or element.
The term "biodistribution" refers to the location of the given particle or particles in a biological entity.
The term "microsphere" refers to an object that is substantially spherical in shape and has a diameter less than 1 millimeter.
The term "glass" refers to a hard, brittle, non-crystalline, inorganic substance, which is usually transparent; glasses are often made by fusing silicates with soda, as described by Webster's New World Dictionary. Ed. Guralnik, D B 1984.
The phrase "time of use" refers to the period during which a microsphere is implanted in a patient or subject.
For purposes of this invention, the chemical elements are identified in accordance with the Periodic Table of the Elements, CAS version, Handbook of Chemistry and Physics, 67th Ed., 1986-87, inside cover.

*Microspheres of the Invention*

The present invention relates to a microsphere, comprising a material selected from the group consisting of polymer and resin; a first radioisotope that emits a therapeutic $\beta$-particle; and a second radioisotope that emits a diagnostic $\gamma$-ray; wherein the atomic number of the first radioisotope is not the same as the atomic number of the second radioisotope, according to the claims. The present invention also relates to the microsphere according to the claims wherein the ratio of the radioactivity of the second radioisotope to the first radioisotope is in the range from about 1:10 to about $1:10^7$ at the time of use.
In certain embodiments, the present invention relates to the microsphere according to the claims, wherein the ratio of the radioactivity of the second radioisotope to the first radioisotope is in the range from about $1:10^2$ to $1:10^6$ at the time of use.
In certain embodiments, the present invention relates to the microsphere according to the claims, wherein the ratio of the radioactivity of the second radioisotope to the first radioisotope is in the range from about $1:10^4$ to $1:10^5$ at the time of use. The present invention relates to the microsphere according to the claims, wherein said material is selected from the group consisting of resin and polymer.
In certain embodiments, the present invention relates to the microsphere according to the claims, wherein the diameter of said microsphere is in the range from about 5-75 micrometers.

In certain embodiments, the present invention relates to the microsphere according to the claims, wherein the diameter of said microsphere is in the range from about 5-500 micrometers.

In certain embodiments, the present invention relates to the microsphere according to the claims, wherein the diameter of said microsphere is in the range from about 10-100 micrometers.

In certain embodiments, the present invention relates to the microsphere according to the claims, wherein the diameter of said microsphere is in the range from about 20-50 micrometers.

In certain embodiments, the present invention relates to the microsphere according to the claims, wherein said microsphere is solid, hollow, or comprises a plurality of hollow cells.

In certain embodiments, the present invention relates to the microsphere according to the claims, wherein said microsphere is solid or hollow.

In certain embodiments, the present invention relates to the microsphere according to the claims, wherein said microsphere is solid.

In certain embodiments, the present invention relates to the microsphere according to the claims, wherein the density of said microsphere is in the range from about 1.0-4.0 grams/cubic centimeter.

In certain embodiments, the present invention relates to the microsphere according to the claims, wherein the density of said microsphere is in the range from about 1.0-3.0 grams/cubic centimeter.

In certain embodiments, the present invention relates to the microsphere according to the claims, wherein the density of said microsphere is in the range from about 1.0-2.0 grams/cubic centimeter.

In certain embodiments, the present invention relates to the microsphere according to the claims, wherein said first radioisotope is not leached from said microsphere to an extent greater than about 3%; wherein said second radioisotope is not leached from said microsphere to an extent greater than about 3%.

In certain embodiments, the present invention relates to the microsphere according to the claims, wherein said first radioisotope is not leached from said microsphere to an extent greater than about 1%; wherein said second radioisotope is not leached from said microsphere to an extent greater than about 1%.

In certain embodiments, the present invention relates to the microsphere according to the claims wherein said first radioisotope is $^{90}$Y or $^{32}$P.

In certain embodiments, the present invention relates to the aforementioned microsphere according to the claims, wherein said first radioisotope is $^{90}$Y.

In certain embodiments, the present invention relates to the microsphere according to the claims wherein said second radioisotope is $^{198}$Au.

In certain embodiments, the present invention relates to the microsphere according to the claims wherein said first radioisotope is $^{90}$Y or $^{32}$P; and said second radioisotope is $^{198}$Au.

In certain embodiments, the present invention relates to the microsphere according to the claims wherein said first radioisotope is $^{90}$Y; and said second radioisotope is $^{198}$Au.

*Methods of the Invention*

The present invention also relates to a method of preparing a radioactive microsphere, comprising the steps of:

combining a non-radioactive precursor of a first radioisotope, a non-radioactive precursor of a second radioisotope, and a material selected from the group consisting of polymer, and resin, to form a mixture; wherein the atomic number of the first radioisotope is not the same as the atomic number of the second radioisotope;

fabricating a microsphere from said mixture; and

bombarding said microsphere with neutrons, according to the claims.

In certain embodiments, the present invention relates to the aforementioned method, wherein said non-radioactive precursor of a first radioisotope is Y; and said non-radioactive precursor of a second radioisotope is Au.

In certain embodiments, the present invention relates to the aforementioned method, wherein the ratio of the radioactivy of the second radioisotope to the first radioisotope is in the range from about 1:10 to about 1:10$^7$.

In certain embodiments, the present invention relates to the aforementioned method, wherein the ratio of the radioactivy of the second radioisotope to the first radioisotope is in the range from about 1:10$^2$ to 1:10$^6$.

In certain embodiments, the present invention relates to the aforementioned method, wherein the ratio of the radioactivy of the second radioisotope to the first radioisotope is in the range from about 1:10$^4$ to 1:10$^5$.

The present invention relates to the aforementioned method, wherein the ratio of the radioactivity of the second radioisotope to the first radioisotope is in the range from about 1:10 to about 1:10$^7$ at the time of use.

In certain embodiments, the present invention relates to the aforementioned method, wherein the ratio of the radioactivy of the second radioisotope to the first radioisotope is in the range from about 1:10$^2$ to 1:10$^6$ at the time of use.

In certain embodiments, the present invention relates to the aforementioned method, wherein the ratio of the radioactivity of the second radioisotope to the first radioisotope is in the range from about $1:10^4$ to $1:10^5$ at the time of use.

In certain embodiments, the present invention relates to the aforementioned method, wherein said first radioisotope is $^{90}Y$ or $^{32}P$,

In certain embodiments, the present invention relates to the aforementioned method, wherein said first radioisotope is $^{90}Y$.

The present invention relates to the aforementioned method, wherein said second radioisotope is $^{198}Au$.

In certain embodiments, the present invention relates to the aforementioned method, wherein said first radioisotope is $^{90}Y$ or $^{32}P$; and said second radioisotope is $^{198}Au$.

In certain embodiments, the present invention relates to the aforementioned method, wherein said first radioisotope is $^{90}Y$; and said second radioisotope is $^{198}Au$.

In certain embodiments, the present invention relates to the aforementioned method, wherein said microspheres are administered using a catheter or a syringe.

In certain embodiments, the present invention relates to the aforementioned method, wherein said microspheres are administered by a catheter.

In a further aspect the invention provides a microsphere for use in medicine in accordance with claim 14.

In a further aspect the invention provides a microsphere for use in treating cancer in accordance with claim 15.

*Exemplification*

The invention now being generally described, it will be more readily understood by reference to the following examples, which are included for purposes of illustration of certain aspects and embodiments of the present invention. Microspheres and methods not encompassed by the scope of the claims do not form part of the invention and are cited as reference examples.

### *Example 1*

*Calculation of Optimal Amount of $^{197}Au$ Required for $^{89}Y$-containing Glass Microsphere*

*Experimental Design*

In the present case, we wish to label radioactive $^{90}Y$-containing glass microspheres with a sufficient amount of $^{198}Au$ so that the microspheres are detectable by a gamma camera. For the purpose of the example, the microspheres are of the same composition as commercial Theraspheres (40% $Y_2O_3$ by weight, or 31% Y), except for the presence of the gold compound. The process is to be carried out by neutron activating glass microspheres that contain the stable isotopes $Y^{89}$ and $Au^{197}$. In this example, we wish to compute the amount of initial $Au^{197}$ that is required, if the desired radioactivity at the time of removal of the sample from the neutron flux is to be 100 mCi of $Y^{90}$ and 1 $\mu$Ci of $Au^{198}$ Per 50 mg of glass microspheres. The neutron capture cross-sections for $Y^{89}$ and $Au^{197}$ are 1.3 barns and 98.8 barns, respectively, and the decay constants for $Y^{90}$ and $Au^{198}$ are $3.01 \times 10^{-6}$ s$^{-1}$ and $2.98 \times 10^{-6}$s$^{-1}$, respectively. Supposing that the neutron flux is $1 \times 10^{14}$cm$^{-2}$s$^{-1}$, the neutron capture constants are computed from Eq 4 to be $1.3 \times 10^{-10}$ s$^{-1}$ for $Y^{89}$ and $9.88 \times 10^{-9}$s$^{-1}$ for $Au^{197}$. For both elements, the neutron capture constant is, to a good approximation, negligible compared to the decay constant. Under this circumstance, and as long as the neutron activation time (t) is less than about $5 \times 10^6$s, Eq 6 is approximated by Eq 8, to within about 5%:

$$k_N = \phi \chi \qquad \text{(Eq 4)}$$

$$\frac{dA^*}{dt} = -k_D A^* \qquad \text{(Eq 5)}$$

$$\frac{-dA^*}{dt} = \frac{k_D k_N A_0}{k_D - k_N} \left( e^{-k_N t} - e^{-k_D t} \right) \qquad \text{(Eq 6)}$$

$$-\frac{dA^*}{dt} \approx k_n A_0 \left(1 - e^{-\frac{kt}{0}}\right) \qquad\qquad (Eq\ 8)$$

Using Eq 8, we calculate that activating the 50 mg of glass microspheres (0.174 mmol of Y) to a radioactivity of 100 mCi will require about 1.05*10<5 > s. Substituting values for the Au and Y isotopes into Eq 8 and forming ratios, and noting that the decay constants are nearly identical, we arrive at the final equation which expresses the ratio of radioactivity to the ratio of the initial amounts of stable isotopes:

For the present example, substituting the desired radioactivity values in Eq 9 gives the starting mole ratio of gold to yttrium as:

$$\frac{\dfrac{d(Au^{198})}{dt}}{\dfrac{d(Y^{90})}{dt}} = \frac{\chi_{Au^{197}} Au_0^{197}}{\chi_{Y^{89}} Y_0^{89}} = \frac{76 Au_0^{197}}{Y_0^{89}} \qquad\qquad (Eq\ 9)$$

In the glass composition, which is 31 % Y by weight, the necessary amount of gold is finally computed to be 91 Parts per billion, by weight.

$$\frac{Au_0^{197}}{Y_0^{89}} = 1.32 \times 10^{-7}\ (\text{mole ratio}) = 2.92 \times 10^{-7}\ (\text{mass ratio})$$

### Example 2

In this example, we assume a glass composition containing 13% Y2O3by weight (or 10% Y), and desire radioactivities of 100 mCi for Y<90 > and 10 [mu]Ci for Au<198 > Per 50 mg of glass microspheres. The other quantities are as for Example 1. A similar computation shows that the glass should contain 291 Ppb of gold, and requires a neutron activation time of 6.10*10<5 > s. Similar calculations may be performed for other proportions of these elements or combinations of other elements in any proportions.

### Example 3

Glass Bead Preparation

The procedures for preparing glass microspheres has been reported previously. See U.S. Pat. No. 5,302,369. In these preparations, glass of varying compositions of Si, Al, K, Mg, Al, Pb, and P2O5 has been prepared using reagent grade chemicals. Batches yielding 50 grams of glass were melted in platinum crucibles in an electric furnace at the approximate temperatures. A typical melting cycle required three hours for batch additions at 1000[deg.] C. and three to four hours to refine the melt at the approximate melting temperature. The crucible containing the melt was quenched in 25[deg.] C. water, after which the resultant glass frit was broken from the crucible and ground to -100 mesh. The -100 mesh glass powder was then slowly fed by a vibrating spatula into an oxygen/propane flame where surface tension pulled the molten particles into spheres. The flow rates of oxygen and propane were adjusted for each glass composition so as to yield the highest fraction of spherical particles. After spheroidizing, the microspheres were wet screened with deionized water, rinsed in acetone and dried.

### Claims

1. A microsphere, comprising

    a material selected from the group consisting of polymer and resin;
    a first radioisotope that emits a therapeutic β-particle; and
    a second radioisotope that emits a diagnostic γ-ray;
    wherein the first radioisotope is selected from the group consisting of $^{90}Y$, $^{99m}Tc$, $^{188}Re$, $^{32}P$, $^{166}Ho$, $^{109}Pb$,

$^{140}$La, $^{153}$Sm, $^{165}$Dy, and $^{169}$Er;
the second radioisotope comprises $^{198}$Au; and
the microsphere exhibits a ratio of the radioactivity of the second radioisotope to the first radioisotope in the range from 1:10 to 1:10$^7$.

2. The microsphere of claim 1, wherein the ratio of the radioactivity of the second radioisotope to the first radioisotope is in the range from 1: 10$^2$ to 1:10$^6$.

3. The microsphere of claim 1, wherein the ratio of the radioactivity of the second radioisotope to the first radioisotope is in the range from 1: 10$^4$ to 1: 10$^5$.

4. The microsphere of claim 1, wherein the material is a polymer.

5. The microsphere of claim 1, wherein the material is a resin.

6. The microsphere of claim 1, wherein the diameter of the microsphere is in the range from 5-500 micrometers.

7. The microsphere of claim 1, wherein the diameter of the microsphere is in the range from 10-100 micrometers.

8. The microsphere of claim 1, wherein the microsphere is solid, hollow, or comprises a plurality of hollow cells.

9. The microsphere of claim 1, wherein the density of the microsphere is in the range from 1.0-4.0 grams/cubic centimeter.

10. The microsphere of claim 1, wherein under mammalian physiological conditions the first radioisotope is not leached from the microsphere to an extent greater than 3%; wherein under mammalian physiological conditions the second radioisotope is not leached from the microsphere to an extent greater than 3%.

11. The microsphere of any one of claims 1-10, wherein the first radioisotope is $^{90}$Y, $^{166}$Ho or $^{188}$Re.

12. A method of preparing a radioactive microsphere, comprising the steps of:

combining a non-radioactive precursor of a first radioisotope, a non-radioactive precursor of a second radioisotope, and a material selected from the group consisting of polymer and resin, to form a mixture;
wherein the first radioisotope is selected from the group consisting of $^{90}$Y, $^{99m}$Tc, $^{188}$Re, $^{32}$P, $^{166}$Ho, $^{109}$Pb, $^{140}$La, $^{153}$Sm, $^{165}$Dy, and $^{169}$Er;
the second radioisotope comprises $^{198}$Au; and the microsphere exhibits a ratio of the radioactivity of the second radioisotope to the first radioisotope in the range from 1:10 to 1:10$^7$;
fabricating a microsphere from the mixture; and
bombarding the microsphere with neutrons.

13. The method of claim 12, wherein the material is polymer and the nonradioactive precursor of a first radioisotope is Y, Ho or Re.

14. A microsphere as claimed in any of claims 1 to 11, for use in medicine.

15. A microsphere as claimed in any of claims 1 to 11, for use in treating cancer.

**Patentansprüche**

1. Mikrokügelchen, umfassend
ein Material, das ausgewählt ist aus der Gruppe, bestehend aus Polymer und Harz;
ein erstes Radioisotop, das ein therapeutisches β-Teilchen emittiert; und
und ein zweites Radioisotop, das eine diagnostische γ-Strahlung emittiert;
wobei das erste Radioisotop ausgewählt ist aus der Gruppe, bestehend aus $^{90}$Y, $^{99m}$Tc, $^{188}$Re, $^{32}$P, $^{166}$Ho, $^{109}$Pb, $^{140}$La, $^{153}$Sm, $^{165}$Dy, und $^{169}$Er;
das zweite Radioisotop $^{198}$Au umfasst; und

das Mikrokügelchen ein Verhältnis der Radioaktivität des zweiten Radioisotops zum ersten Radioisotop im Bereich von 1:10 bis 1:10$^7$ aufweist.

2. Mikrokügelchen nach Anspruch 1, wobei das Verhältnis der Radioaktivität des zweiten Radioisotops zum ersten Radioisotop im Bereich von etwa 1:10$^2$ bis 1:10$^6$ liegt.

3. Mikrokügelchen nach Anspruch 1, wobei das Verhältnis der Radioaktivität des zweiten Radioisotops zum ersten Radioisotop im Bereich von etwa 1:10$^4$ bis 1:10$^5$ liegt.

4. Mikrokügelchen nach Anspruch 1, wobei das Material ein Polymer ist.

5. Mikrokügelchen nach Anspruch 1, wobei das Material ein Harz ist.

6. Mikrokügelchen nach Anspruch 1, wobei der Durchmesser des Mikrokügelchens im Bereich von etwa 5 bis 500 Mikrometern liegt.

7. Mikrokügelchen nach Anspruch 1, wobei der Durchmesser des Mikrokügelchens im Bereich von 10 bis 100 Mikrometern liegt.

8. Mikrokügelchen nach Anspruch 1, wobei das Mikrokügelchen massiv oder hohl ist oder eine Vielzahl an hohlen Zellen umfasst.

9. Mikrokügelchen nach Anspruch 1, wobei die Dichte des Mikrokügelchens im Bereich von 1,0 bis 4,0 Gramm/Kubikzentimeter beträgt.

10. Mikrokügelchen nach Anspruch 1, wobei unter physiologischen Säugetierbedingungen das erste Radioisotop nicht in einem Ausmaß von mehr als 3 % aus dem Mikrokügelchen austritt; wobei unter physiologischen Säugetierbedingungen das zweite Radioisotop nicht in einem Ausmaß von mehr als 3 % aus dem Mikrokügelchen austritt.

11. Mikrokügelchen nach einem der Ansprüche 1 bis 10, wobei das erste Radioisotop $^{90}$Y, $^{166}$Ho oder $^{188}$Re ist.

12. Verfahren zur Herstellung eines radioaktiven Mikrokügelchens, umfassend die folgenden Schritte:

Kombinieren eines nicht radioaktiven Vorläufers eines ersten Radioisotops, eines nicht radioaktiven Vorläufers eines zweiten Radioisotops, und eines Materials, das ausgewählt ist aus der Gruppe, bestehend aus Polymer und Harz, zur Bildung einer Mischung;
wobei das erste Radioisotop ausgewählt ist aus der Gruppe, bestehend aus $^{90}$Y, $^{99m}$Tc, $^{188}$Re, $^{32}$P, $^{166}$Ho, $^{109}$Pb, $^{140}$La, $^{165}$Dy, und $^{169}$Er;
das zweite Radioisotop $^{198}$Au umfasst; und das Mikrokügelchen ein Verhältnis von Radioaktivität des zweiten Radioisotops zu dem ersten Radioisotop in dem Bereich von 1:10 bis 1:10$^7$ aufweist;
Erzeugen eines Mikrokügelchens aus der Mischung; und
Beschießen des Mikrokügelchens mit Neutronen.

13. Verfahren nach Anspruch 12, wobei das Material Polymer ist und der nichtradioaktive Vorläufer des ersten Radioisotops Y, Ho oder Re ist.

14. Mikrokügelchen nach einem der Ansprüche 1 bis 11, zur Verwendung in der Medizin.

15. Mikrokügelchen nach einem der Ansprüche 1 bis 11 zur Verwendung bei der Behandlung von Krebs.

**Revendications**

1. Microsphère, comprenant
un matériau choisi parmi le groupe constitué d'un polymère et d'une résine ;
un premier radio-isotope qui émet une particule β thérapeutique ; et
un second radio-isotope qui émet un rayonnement γ diagnostique ;
dans lequel le premier radio-isotope est choisi parmi le groupe constitué de $^{90}$Y, $^{99m}$Tc, $^{188}$Re, $^{32}$P, $^{166}$Ho, $^{109}$Pb,

$^{140}$La, $^{153}$Sm, $^{165}$Dy et $^{169}$Er ;
le second radio-isotope comprend $^{198}$Au ; et
la microsphère présente un rapport de radioactivité entre le second radio-isotope et le premier radio-isotope dans la plage de 1:10 à 1:10$^7$.

2. Microsphère selon la revendication 1, dans laquelle le rapport de radioactivité entre le second radio-isotope et le premier radio-isotope est dans la plage de 1:10$^2$ à 1:10$^6$.

3. Microsphère selon la revendication 1, dans laquelle le rapport de radioactivité entre le second radio-isotope et le premier radio-isotope est dans la plage de 1:10$^4$ à 1:10$^5$.

4. Microsphère selon la revendication 1, dans laquelle le matériau est un polymère.

5. Microsphère selon la revendication 1, dans laquelle le matériau est une résine.

6. Microsphère selon la revendication 1, dans laquelle le diamètre de la microsphère est dans la plage de 5 à 500 micromètres.

7. Microsphère selon la revendication 1, dans laquelle le diamètre de la microsphère est dans la plage de 10 à 100 micromètres.

8. Microsphère selon la revendication 1, dans laquelle la microsphère est solide, creuse, ou comprend une pluralité de cellules creuses.

9. Microsphère selon la revendication 1, dans laquelle la densité de la microsphère est dans la plage de 1,0 à 4,0 grammes/centimètre cube.

10. Microsphère selon la revendication 1, dans laquelle, dans des conditions physiologiques de mammifère, le premier radio-isotope n'est pas lixivié de la microsphère selon une mesure supérieure à 3 % ; dans lequel dans des conditions physiologiques de mammifère, le second radio-isotope n'est pas lixivié de la microsphère selon une mesure supérieure à 3 %.

11. Microsphère de l'une quelconque des revendications 1 à 10, dans laquelle le premier radio-isotope est $^{90}$Y, $^{166}$Ho ou $^{188}$Re.

12. Procédé de préparation d'une microsphère radioactive, comprenant les étapes consistant à :

combiner un précurseur non radioactif d'un premier radio-isotope, un précurseur non radioactif d'un second radio-isotope et un matériau choisi parmi le groupe constitué d'un polymère et d'une résine, pour former un mélange ;
dans lequel le premier radio-isotope est choisi parmi le groupe constitué de $^{90}$Y, $^{99m}$Tc, $^{188}$Re, $^{32}$P, $^{166}$Ho, $^{109}$Pb, $^{140}$La, $^{153}$Sm, $^{165}$Dy et $^{169}$Er ;
le second radio-isotope comprend $^{198}$Au ; et la microsphère présente un rapport de radioactivité entre le second radio-isotope et le premier radio-isotope dans la plage de 1:10 à 1:10$^7$ ;
fabriquer une microsphère à partir du mélange ; et
bombarder la microsphère à l'aide de neutrons.

13. Procédé selon la revendication 12, dans lequel le matériau est un polymère et le précurseur non radioactif d'un premier radio-isotope est Y, Ho ou Re.

14. Microsphère selon l'une quelconque des revendications 1 à 11, destinée à être utilisée en médecine.

15. Microsphère selon l'une quelconque des revendications 1 à 11, destinée à être utilisée pour le traitement d'un cancer.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6149889 A
- US 6328700 B
- US 5302369 A

### Non-patent literature cited in the description

- *Langenbeck's Arch. Surg.,* 1999, 313-327
- **HÄFELI U O ; CASILLAS S ; DIETZ D W ; PAUER G J ; RYBICKI L A ; CONZONE S D ; DAY D E.** Hepatic tumor radioembolization in a rat model using radioactive rhenium (186Re/188Re) glass microspheres. *Int. J. Radiation Oncology Biol. Phys.,* 1999, vol. 44, 189-199
- **LINK K H ; KOMNMAN M. ; FORMENTINI A ; LEDER G ; SUNELAITIS E ; SCHATZ M ; PRELMAR J ; BEGER H G.** Regional chemotherapy of non-resectable liver metastases from colorectal cancer-literature and institutional review. *Langenbeck's Arch. Surg.,* 1999, vol. 384, 344-353
- **BASTIAN P ; BARTKOWSKI R ; KOHLER H ; KISSEL T.** Chemo-embolization of experimental liver metastases. Part 1: distribution of biodegradable microspheres of different sizes in an animal model for the locoregional therapy. *Eur. J. Pharm. Biopharm.,* 1998, vol. 46, 243-254
- **ACKERMAN N B ; LIEN W M ; KONDI E S ; SILVERMAN N A.** The blood supply of experimental liver metastases. The distribution of hepatic artery and portal vein blood to "small" and "large" tumors. *Surgery,* 1969, vol. 66, 1067-1072
- **NOLAN et al.** Intravascular Particulate Radioisotope Therapy. *The American Surgeon,* 1969, vol. 35, 181-188
- **GRADY et al.** Intra-Arterial Radioisotopes to Treat Cancer. *American Surgeon,* 1960, vol. 26, 678-684
- **ZIELINSKI ; KASPRZYK.** Synthesis and Quality Control Testing of P labelled Ion Exchange Resin Microspheres for Radiation Therapy of Hepatic Neoplasms. *Int. J. Appl. Radiat. Isot.,* 1983, vol. 34, 1343-1350
- **HO S ; LAU W Y ; LEUNG T W T ; CHAN M ; NGAR Y K ; JOHNSON P J ; LI A K C.** Clinical evaluation of the partition model for estimating radiation doses from yttrium-90 microspheres in the treatment of hepatic cancer. *Eur. J. Nucl. Med.,* 1997, vol. 24, 293-298
- **MUMPER R J ; RYO U Y ; JAY M.** Neutron activated holmium-166-Poly(L-lactic acid) microspheres: A potential agent for the internal radiation therapy of hepatic tumours. *J. Nucl. Med.,* 1991, vol. 32, 2139-2143
- **TURNER J H ; CLARINGBOLD P G ; KLEMP P F B ; CAMERON P J ; MARTINDALE A A ; GLANCY R J ; NORMAN P E ; HETHERINGTON E L ; NAJDOVSKI L ; LAMBRECHT R M.** Ho-microsphere liver radiotherapy: a preclinical SPECT dosimetry study in the pig. *Nucl. Med. Comm.,* 1994, vol. 15, 545-553
- **HO S ; LAU W Y ; LEUNG T W T ; JOHNSON P J.** Internal radiation therapy for patients with primary or metastatic hepatic cancer. *Cancer,* 1998, vol. 83, 1894-1907
- **SPENCER R P.** Applied principles of radiopharmaceutical use in therapy. *Nucl. Med. Biol.,* 1986, vol. 13, 461-463
- **SPENCER R P.** Short-lived radionuclides in therapy. *Nucl. Med. Biol.,* 1987, vol. 14, 537-538
- **CONZONE S D ; HÄFELI U O ; DAY D E ; EHRHARDT G J.** Preparation and properties of radioactive rhenium glass microspheres intended for in vivo radioembolization therapy. *J. Biomed. Mater. Res.,* 1998, vol. 42, 617-625
- **HO S ; LAU W Y ; LEUNG T W T ; CHAN M ; NGAR Y K ; JOHNSON P J ; LI A K C.** Clinical evaluation of the partition model for estimating radiation doses from yttrium-90 microspheres in the treatment of hepatic cancer. *Eur. J Nucl. Med.,* 1997, vol. 24, 293-298
- **MUMPER R J ; RYO U Y ; JAY M.** Neutron activated holmium-166-Poly(L-lactic acid) microspheres: A potential agent for the internal radiation therapy of hepatic tumours. *J. Nucl. Med.,* 1991, vol. 32, 2139-2143
- **HÄFELI U O ; CASILLAS S ; DIETZ D W ; PAUER G J ; RYBICKI L A ; CONZONE S D ; DAY D E.** Hepatic tumor radioembolization in a rat model using radioactive rhenium (186Re/188Re) glass microspheres. *Int. J. Radiation Oncology Biol. Phys.,* 1999, vol. 44, 189-199

- CONZONE S D ; HÄFELI U O ; DAY D E ; EHRHARDT G J. Preparation and properties of radioactive rhenium glass microspheres intended for in vivo radioembolization therapy. *J. Biomed. Mater. Res.,* 1998, vol. 42, 617-625
- BROWN R F ; LINDESMITH L C ; DAY D E. 166-Holmium-containing glass for internal radiotherapy of tumors. *Int. J. Rad. Appl. Instrum. B,* 1991, vol. 18, 783-790
- KAWASHITA M ; MIYAJI F ; KOKUBO T ; TAKAOKA G H ; YAMADA I ; SUZUKI Y ; INOUE M. Surface structure and chemical durability of P<+> - implanted Y2O3-Al2O3-SiO2 glass for radiotherapy of cancer. *J. Non-Cryst. Solids,* 1999, vol. 255, 140-148
- SCHUBIGER P A ; BEER H-F ; GEIGER L ; RÖSLER H ; ZIMMERMAN A ; TRILLER J ; METTLER D ; SCHILT, W. Y-resin particles-animal experiments on pigs with regard to the introduction of superselective embolization therapy. *Nucl. Med. Biol.,* 1991, vol. 18, 305-311
- ZIMMERMAN A ; SCHUBIGER P A ; METTLER D ; GEIGER L ; TRILLER J ; RÖSLER H. Renal pathology after arterial yttrium-90 microsphere administration in pigs. A model for superselective radioembolization therapy. *Invest. Rad.,* 1995, vol. 30, 716-723
- WANG S-J ; LIN W-Y ; CHEN M.-N ; CHI C-S ; CHEN J-T ; HO W-L ; HSIEH B-T ; SHEN L-H ; TSAI Z-T ; TING G. Intratumoral injection of rhenium-18 8microspheres into an animal model of hepatoma. *J Nucl Med.,* 1998, vol. 39, 1752-1757
- LAU W Y ; LEUNG W T ; HO S ; LEUNG N W Y ; CHAN M ; LIN J ; METREWELI C ; JOHNSON P ; LI A K C. Treatment of inoperable hepatocellular carcinoma with intrahepatic arterial yttrium-90 microspheres: a phase I and II study. *Br. J. Cancer,* 1994, vol. 70, 994-999
- LAU W Y ; HO S ; LEUNG T W T ; CHAN M ; HO R ; JOHNSON P J ; LI A K C. Selective internal radiation therapy for nonresectable hepatocellular carcinoma with intraarterial infusion of yttrium microspheres. *Int. J. Radiation Oncology Biol. Phys.,* 1998, vol. 40, 583592
- WUNDERLICH G ; PINKERT J ; ANDREEFF M ; STINTZ M ; KNAPP F F ; KROPP J ; FRANKE W G. Preparation and biodistribution of rhenium-188 labeled albumin microspheres B 20: a promising new agent for radiotherapy. *Appl. Radiat. Isotopes,* 2000, vol. 52, 63-68
- RHODES B A ; ZÖLLE I ; BUCHANAN J W ; WAGNER H N. Radioactive albumin microspheres for studies of the pulmonary circulation. *Radiology,* 1969, vol. 92, 1453-1460
- WATANABE N ; ORIUCHI N ; ENDO K ; INOUE T ; TANADA S ; MURATA H ; SASAKI Y. Yttrium-90 labeled human macroaggregated albumin for internal radiotherapy: combined use with DTPA. *Nucl. Med. Biol.,* 1999, vol. 26, 847-851
- MUMPER R J ; JAY M. Poly(L-lactic acid) microspheres containing neutron-activatable holmium-165: A study of the physical characteristics of microspheres before and after irradiation in a nuclear reactor. *Pharm. Res.,* 1992, vol. 9, 149-154
- HÄFELI U O ; SWEENEY S M ; BERESFORD B A ; HUMM J L ; MACKLIS R M. Effective targeting of magnetic radioactive Y-microspheres to tumor cells by an externally applied magnetic field. Preliminary in vitro and in vivo results. *Nucl. Med. Biol.,* 1995, vol. 22, 147-155
- O'DONNELL P B ; MCGINITY J W. Preparation of microspheres by solvent evaporation technique. *Adv. Drug Del. Rev.,* 1997, vol. 28, 25-42
- GRAY B N ; BURTON M A ; KELLEHER D ; KLEMP P ; MATZ L. Tolerance of the liver to the effects of yttrium-90 radiation. *Int. J. Radiation Oncology Biol. Phys.,* 1990, vol. 18, 619-623
- TIAN J-H ; XU B-X ; ZHANG J-M ; DONG B-W ; LIANG P ; WANG X-D. Ultrasoundguided internal radiotherapy using yttrium-90-glass microspheres for liver malignancies. *J. Nucl. Med.,* 1996, vol. 37, 958-963
- HO S ; LAU W Y ; LEUNG T W T ; CHAN M ; CHAN K W ; LEE W Y ; JOHNSON P J ; LI A K C. Tumour-to-normal ratio of Y microspheres in hepatic cancer assessed with Tc macroaggregated albumin. *Brit. J. Rad.,* 1997, vol. 70, 823-828
- YORKE E D ; JACKSON A ; FOX R A ; WESSELS B W ; GRAY N. Can current models explain the lack of liver complications in Y-90 microsphere therapy?. *Clin. Cancer Res.,* 1999, vol. 5, 3024s-3030s
- INGOLD J ; REED G ; KAPLAN H ; BAGSHAW M. Radiation hepatitis. *Am. J. Roentgenol. Radium Ther. Nucl. Med.,* 1965, vol. 93, 200-208
- VAN ES R J J ; FRANSSEN O ; DULLENS H F J ; BEMSEN M R ; BOSMAN F ; HENNINK W E ; SLOOTWEG P J. The VX2 carcinoma in the rabbit auricle as an experimental model for intra-arterial embolization of head neck squamous cell carcinoma with hydrogel dextran microspheres. *Lab. Anim.,* 1999, vol. 33, 175-184
- TOMURA N ; KATO K ; HIRANO H ; HIRANO Y ; WATARAI J. Chemoembolization of maxillary tumors via the superficial temporal artery using a coaxial catheter system. *Radiation Med.,* 1998, vol. 16, 157
- ARIEL I M ; PADULA G. Irradiation of the spleen by the intra-arterial administration of yttrium microspheres in patients with malignant lymphoma. *Cancer,* 1972, vol. 31, 90-96
- Webster's New World Dictionary. 1984
- Handbook of Chemistry and Physics. 1986